Europäisches Patentamt

European Patent Office

Office européen des brevets

(19)

(11) Publication number: **0 379 788**
**A1**

# EUROPEAN PATENT APPLICATION

(21) Application number: **89311851.3**

(22) Date of filing: **16.11.89**

(51) Int. Cl.5: **A61M 39/02**

(30) Priority: **28.12.88 US 291715**

(43) Date of publication of application:
**01.08.90 Bulletin 90/31**

(84) Designated Contracting States:
**BE DE FR GB**

(71) Applicant: **BAXTER INTERNATIONAL INC. (a Delaware corporation)**
**One Baxter Parkway**
**Deerfield Illinois 60015(US)**

(72) Inventor: **Richardson, James C.**
**714 Leslie Lane**
**Schaumburg, IL 60194(US)**

(74) Representative: **MacGregor, Gordon et al**
**ERIC POTTER & CLARKSON St. Mary's Court**
**St. Mary's Gate**
**Nottingham, NG1 1LE(GB)**

(54) Improved injection site.

(57) Disclosed is an apparatus for introducing supplemental medicament fluids into a medical fluid stream in medical fluid administration system, comprising a housing (32) having an inlet (34) and outlet (36) for the medical fluid and at least one aperture (38) defining a supplemental fluid passage. The aperture (38) has a sealing plug (48) and a protective cover (58) over the sealing plug (48), which is removably affixed to the housing (32), with a microporous filter (56) interposed between the sealing plug (48) and the protective covering (58). The microporous filter (56) is gas permeable and substantially microbe impermeable. The protective covering is configured to permit fluid communication with the filter member, and to be taper and prior use indicative.

FIG. 1

FIG. 2

## IMPROVED INJECTION SITE

### TECHNICAL FIELD

This invention generally relates to injection sites in a medical fluid infusion system, and, more specifically relates to injection site which provide a tamper or use indication.

### BACKGROUND OF THE INVENTION

Infusion of medical fluid into a patient, such as infusion of parenteral solutions, occurs when a patient is in a physically critical condition, for example, after severe blood loss or during surgery. It is common in these situations that multiple medicaments are added to the fluid stream, for example, several antibiotics and/or heart stimulants. The medications are preferably added to the established fluid access to avoid any delay associated with establishing a new venous or arterial access site in the patient. Use of the same access site also, of course, avoids the need for multiple injection sites in a given patient.

Injection sites have previously been used in medical fluid administration systems for the purpose of injecting a supplemental medication, or adding a supplemental fluid to the established medical fluid stream. Such injection sites usually are in the form of a V or a Y, with a resealable septum on one branch, through which the supplemental medication is added. Typically, these types of injection sites must be sterilized before use. Sterilization is usually accomplished by wiping the injection site septum with alcohol or other sterilizing fluid.

It may also be desirable in some applications for a medical staff member to be able to determine if an injection site has been tampered with or previously used. Certain medicaments, for example, when mixed together, may result in chemical reactions which can cause blockages in the parenteral supply tubes at or near the injection site. This may occur, for example, when one injection of one medication is immediately followed by an injection of a non-compatible medication, before the medical fluid stream has fully dissipated the first medicament.

One form of an injection site with a tamper indicator cover is described in U.S. Patent No. 4,430,077. This patent discloses an injection site on a medical fluid delivery tube, with a cap covering the injection site. The injection site cannot be used until the cap is removed, and, once the cap is removed, the cap cannot be replaced. Therefore, a quick visual inspection will reveal whether the injection site was tampered with or previously used. To allow initial sterilization during production, a small opening is provided between the cap and the septum to permit the entry of ethylene oxide gas during manufacture.

Although the injection site of the above-cited patent indicates prior use, it also requires relatively precise molding. Furthermore, after the initial sterilization or after the primary package is opened and ambient air enters the opening between the cap and septum, the septum is no longer sterile.

Accordingly, it is a general object of the present invention to provide means for injecting medicaments and the like which does not suffer from the drawbacks described above.

It is a more particular object of this invention to provide an injection site with tamper and prior use indicative covers.

These and other objects of the present invention are set forth in the following detailed description of the illustrated embodiment of the present invention.

### SUMMARY OF THE INVENTION

The present invention is directed to an apparatus for introducing supplemental medicament fluids into a medical fluid stream in a medical fluid administration system. The apparatus comprises a housing having an inlet and an outlet for the medical fluid and at least one aperture defining a supplemental fluid passage. The aperture has sealing means, such as a pierceable, resealable septum or plug, for sealing the supplemental fluid passage. A cover over the sealing plug is removably affixed to the housing with a microporous filter interposed between the sealing plug and the cover. The microporous filter is gas permeable and substantially microbe impermeable. The cover is configured to permit gas communication with the filter member for sterilization of the plug.

In accordance with a further aspect of the present invention, the cap includes a first portion which covers the aperture and a second portion affixed to the housing. A frangible section joins the two portions so that upon removal of the first portion, to obtain access to the plug, the second portion remains attached to the housing so as to indicate that the injection site has been used or that tampering has occurred.

## DESCRIPTION OF THE DRAWINGS

Figure 1 is a vertical plan view of a medical fluid administration apparatus embodying the present invention;

Figure 2 is a perspective view of a multiport injection site manifold in accordance with this invention, with one injection site shown in an exploded view and partially in section;

Figure 3 is a top view of the multiport injection site manifold of Figure 2, with all of the caps removed;

Figure 4 is a side view of the multiport injection site manifold of Figure 2, with all of the caps removed;

Figure 5 is a end view of the multiport injection site manifold of Figure 2, with one cap on and one cap off;

Figure 6 is a cross-sectional side view of the multiport injection site manifold taken along line 6-6 of Figure 3;

Figure 7 is a top view of an injection site cap according to the present invention;

Figure 8 is a side view of the cap of Figure 7;

Figure 9 is a cross-sectional view of the cap of Figure 7, taken along line 9-9;

Figure 10 is an illustration of a cover being lifted off of the manifold;

Figure 11 is an illustration of an injection site in use, with an infusion needle shown in phantom lines.

## DETAILED DESCRIPTION OF THE PREFERRED EMBODIMENT

The invention, together with the objects and advantages thereof, may best be understood by reference to the following description, taken in conjunction with the accompanying drawings; wherein the several figures of which like reference characters identify identical elements.

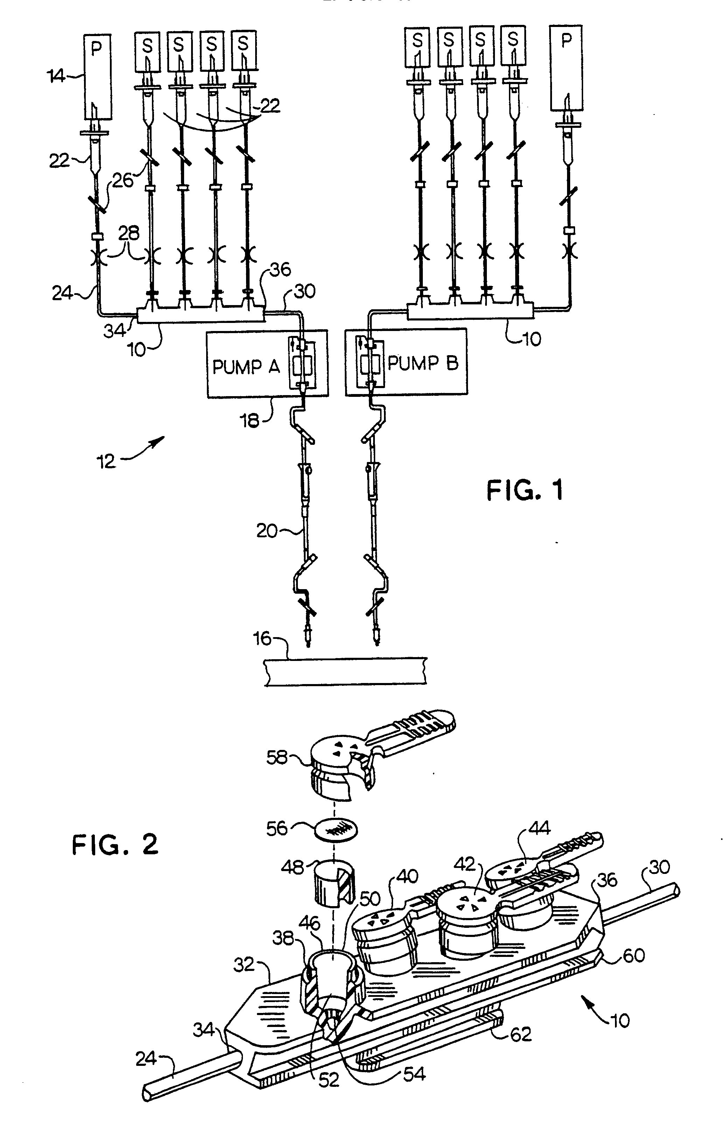

Referring to Figure 1, the present invention, for purposes of illustration only, may be generally embodied in a medical delivery system 12 including a medical solution bag 14, a manifold according to this invention 10, an infusion pump 18 and a patient delivery tube 20. Of course, the present invention is not limited to the illustrated medical fluid delivery system, and the scope of the invention is set forth in the appended claims.

The medical solution delivery system 12 also includes a bag access means, such as a spike or piercing pin 22, which is used to puncture the access port on solution bag 14 as is well known in the field. For purposes of illustration only, the bag 14 may contain a parenteral solution and is thus labeled "P". Of course, the present invention is not limited to the administration of parenteral solutions. A first tube 24 is connected between the bag access means 22 and the multiport injection site manifold 10 embodying the present invention, which can be blocked via a tube clip 26 as is known in the art, or an occluder or flow control clamp 28 (shown in graphic form) as is also known in the art. The multiport injection site manifold 10 is connected to the infusion pump 18 via a second tube 30. The infusion pump 18 provides a constant rate of fluid flow through the delivery tube 20.

The multiport injection site manifold 10 provides a means for introducing supplemental medicaments into the parenteral solution administration system. In Figure 1, four supplemental medicaments, labeled "S" are shown connected to the multiport injection site manifold 10 of the present invention. While four injection sites are shown on this manifold 10, it is to be understood that this is for diagrammatic illustration only, and that a manifold according to this invention could comprise one or more such supplemental injection sites. Each supplemental medicament S is attached to a bag piercing means 22 and a delivery tube as is known in the art. The tubes can be blocked by tube clips or flow control clamps, as is well known in the art.

If necessary, multiple parenteral delivery systems 12 may be attached to the patient-recipient 16 at one time. In Figure 1, two parenteral delivery systems are shown, each system having a parenteral solution bag P and four supplemental medicaments S attached to a multiport injection site manifold 10 according to this invention.

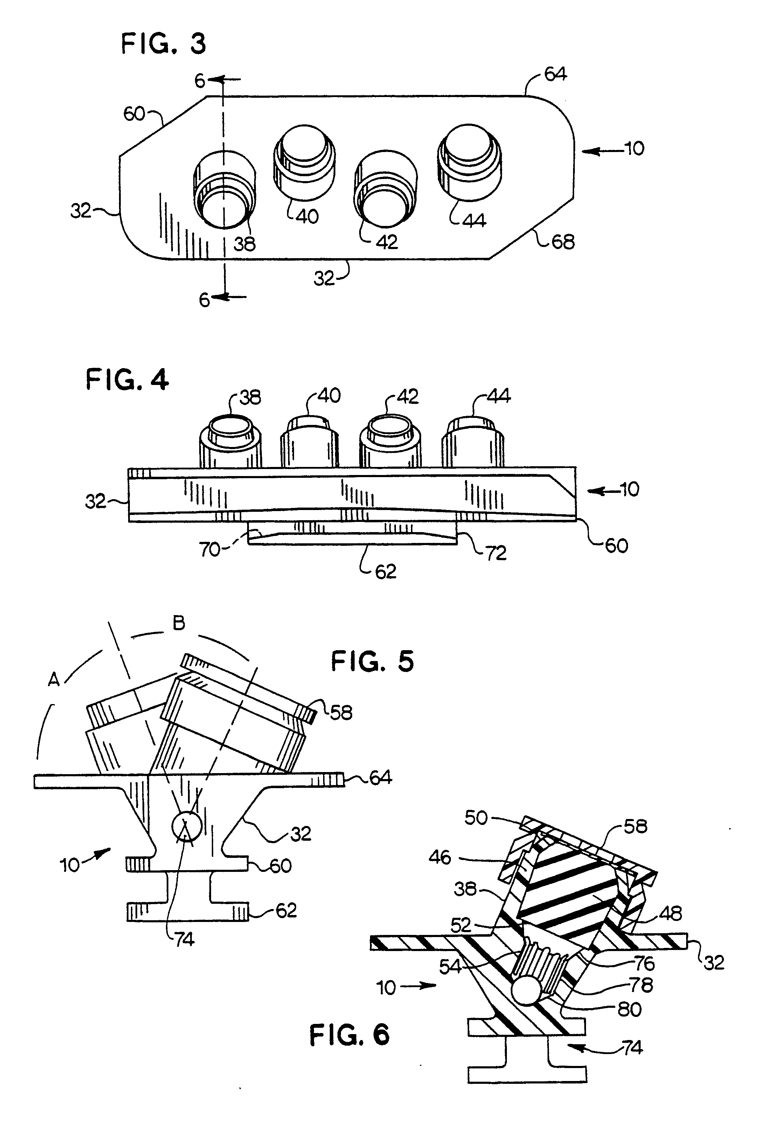

Turning now to Figure 2, a multiport injection site manifold 10 is shown in perspective view, with one injection site shown in exploded view and partially cut-away to show the details of the invention. A housing 32 is connected to the first tube 24 at an inlet 34 and to the second tube 30 at an outlet 36. The housing 32 including a medical fluid passage (not shown in Figure 2, but discuss herein below in connection with Figures 3, 5 and 6) effecting fluid communication between the inlet 34 and the outlet 36. The housing of this preferred embodiment has four apertures defining supplemental fluid passages, 38, 40, 42, and 44 forming a manifold according to this invention. The supplemental fluid passages are identical in configuration and will therefore be described in connection with supplemental fluid passage 38.

The housing 32 is molded in one piece of rigid thermoplastic material or suitable substitute. The preferred material is rigid polyvinylchloride. Other materials and methods of construction could be used without departing from the spirit of this invention, for example, holding the housing in several pieces and cementing the pieces together.

The supplemental fluid passage 38 comprises a cylindrical portion 46 attached to and integral with the housing 32. The cylindrical portion 46 provides a means for receiving and holding a sealing plug or septum 48. The sealing plug 48 in this embodiment of this invention is a needle-pierceable, self-sealing solid cylinder which provides needle-access to the fluid passage within the housing 32, and also seals and supplemental fluid passage when the access needle is withdrawn. In this embodiment, the sealing plug 48 is rubber, as is well known in the art, but the invention is not limited to this material and other materials, such as latex, may be appropriately used. The bottom of the sealing plug 48 is in sealing contact with a plug seat 52, more fully illustrated in connection with Figure 6, which forms along with the plug 48 a fluid-tight seal in the supplemental fluid passage 38. Below the plug seat 52 is a tapered, fluted channel 54 which provides guidance of an access needle into the fluid passage in the housing 32, and provides fluid communication between the medical fluid passage in the housing 32 and the supplemental fluid passage 38. The fluted channel 54 will be described hereinafter below, in connection with Figure 6.

In Figure 2 there is also illustrated a filter member 56 and a cap 58. The filter 56 is a gas permeable, microbe impermeable microporous filter. In this embodiment, the filter 56 is preferably made of a commercially available Tyvec brand material. The filter 56 as will be described herein below, acts as a microbe barrier while simultaneously permitting sterilizing gas to sterilize the surface of the sealing plug and creating a sterile region between the sealing plug 48 and the filter 56. The cap 58 surmounts the cylindrical portion 38 of the injection site and covers the sealing plug 48, forming a sterile system with the added advantage, as will be described herein below, of being tamper and use indicative.

On the bottom of the housing 32 is a first flange 60 and a second flange 62 for mounting the multiport injection site manifold 10 on, for example, an infusion pump 18. The flanges, in the preferred embodiment, are formed integrally with the housing 32.

Turning now to Figure 3, a top view of the multiport injection site manifold 10 is shown with the caps 58 removed. As stated above, there are four supplemental fluid passages 38, 40, 42 and 44 on the housing 32 of this invention, in the preferred embodiment. The illustration of Figure 3 shows a staggered orientation among the injection site 38, 40, 42 and 44. Each supplemental fluid passage is angled to the opposite side of the adjacent supplemental fluid passage, as best seen in Figures 3, 4 and 5, to provide the user with better access to a desired supplemental fluid passage, for example, when the other supplemental fluid passages are in use. Each supplemental fluid passage is at an acute angle A of approximately 67° to the plane of the top portion 64 of the housing 32, and at an acute angle B of 45° angle to each other, in this preferred embodiment. The supplemental fluid passages are molded in one piece with the housing 32 in the preferred embodiment.

The top portion 64 of the housing 32, in the preferred embodiment, is rectangular or oval in shape, with diagonally opposite corners removed along approximately parallel chords resulting in two flat edges 66, 68. These flat edges can, for example, aid in providing finger holds or gripping surfaces for inserting the flange 62 onto a holding device of an infusion pump 18. Other housing 32 shapes could be utilized without departing from the spirit of this invention.

Turning now to Figure 4, a side elevation is shown, with the caps 58 removed. In Figure 4, the flanges 60 and 62 on the bottom of the manifold 10 are clearly illustrated. The lower flange 62 has ramps 70, 72 at each end to aid in insertion of the flange 64 onto a holding device (not shown) as would be found on an infusion pump.

Turning now to Figure 5, an end elevation of a multiport injection site manifold 10 is shown, with a cap 58 on and one cap off. The housing 32 in this preferred embodiment tapers from the top portion 64 substantially towards the first flange or plate 60, in a "V" shape. In this embodiment, the top portion 64 provides the user with a fingerhold for the manifold, whereas the remainder of the housing is narrower to allow gripping. As described earlier, the first flange 60 and second flange 62 provide a means for frictional fit onto a holding device for mounting the manifold 10. The medical fluid passage 74, which extends the full length of the manifold is also shown in Figure 5.

Turning now to Figure 6, an end cross-sectional view of the manifold 10 is shown, taken along line 6-6 of Figure 3, illustrating the intersection of the medical fluid passage 74 and the supplemental fluid passage 38. In this preferred embodiment, the supplemental fluid passage 38 is at approximately 90° to the medical fluid passage 74, but a greater or lessor angle, for example a V or Y angle, could be used in a different application or embodiment.

The tapered and fluted channel 54 is at the intersection of the medical fluid passage 74 and the supplemental fluid passage 38 in this embodiment. The fluted channel 54 includes a substantially tapered conical top portion 76, a substantially cylindrical fluted portion 78 and substantially conical bottom portion 80 where the fluted channel 54 joins the medical fluid passage 74. The fluted channel 54 includes a plurality of axial splines located on the surface in the cylindrical fluted portion 78. The tapered fluted passage 54 acts as a transitional passage from the sealing plug 48 to the parenteral fluid passage 74, and acts as a needle guide into the medical fluid passage 74.

The sealing plug 48 is shown engaged in the supplemental fluid passage 38 between the plug seat 52 and retaining ring 50. Retaining ring 50 is formed by heat-swaging the upper end of the supplemental fluid passage wall into a tight compressive and sealing engagement with plug 48. This results in an axial compression of the plug and induces radial compression by the walls of the fluid passage 38 which form an airtight fluid seal. The plug seat 52 is an annular ring formed from the housing which provides a shoulder that sealingly engages the bottom of the sealing plug 48. The retaining ring 50 which retains the sealing plug 48 within the supplemental fluid passage 38 may also be a separate piece, preferably manufactured from the same material as the housing in this preferred embodiment, and bonded in place after the sealing plug 48 is inserted into the supplemental fluid passage 38.

A cap 58 of the present invention is shown in Figure 6 attached to an outer side of the cylindrical portion 46. The cap 58, in the preferred embodiment, is manufactured of a flexible formulation of polyvinylchloride, to enhance bonding to the cylindrical portion 46, as is well known in the art.

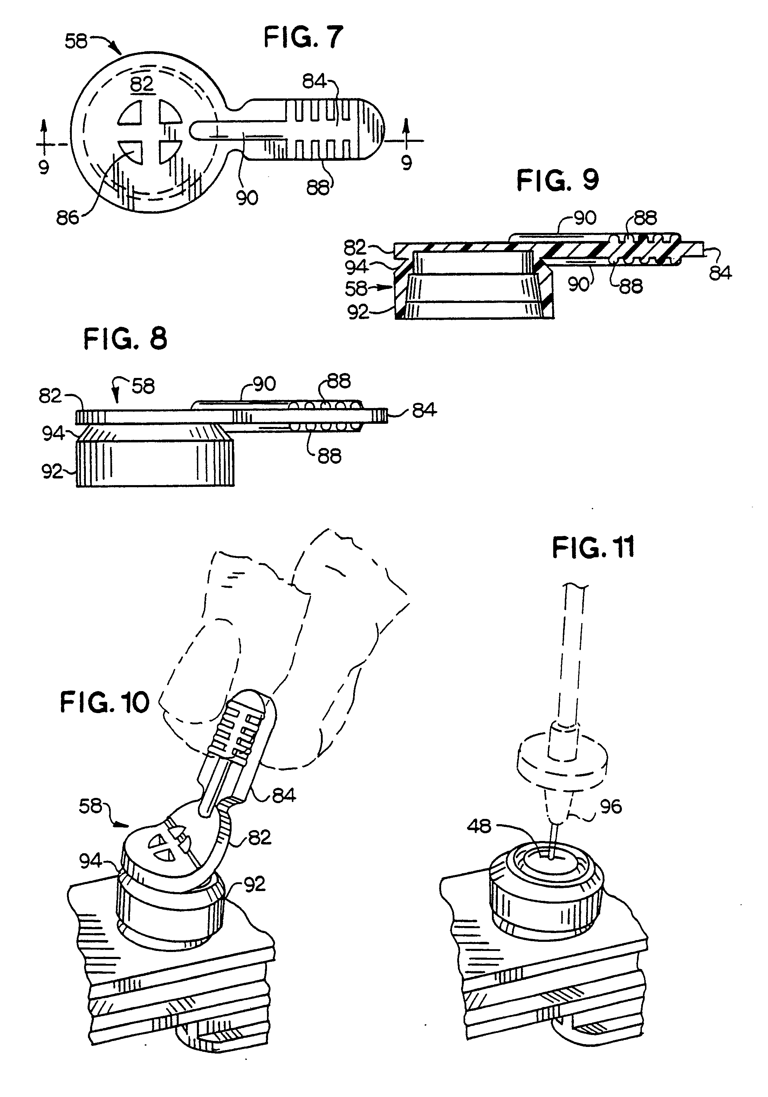

Turning now to Figure 7, a top view of a cap 58 according to the present invention is shown. The cap 58 includes a cover portion 82, and a handle 84. The cover portion 82 completely covers, in this embodiment, the top of the sealing plug 48 and the filter member 56, except for one or more apertures, such as the four pie-shaped openings 86 illustrated in the cover portion 82. The openings 86 permit sterilizing gas to pass through the cap 58, as will be described below. The shape of the openings 84 is shown for illustration purposes only, as any shape openings will work in the present invention provided it allows the passage of sterilizing gas.

The handle 84 provides a fingerhold for lifting the protective cover 82 off of the supplemental fluid passage 38, so that the sealing plug 48 is accessible. To this end, ribs 88 are provided on the handle 84 as a gripping aid. A gusset 90 extends from the ribs 88 to the cover 82, in this embodiment, to strengthen the connection between the handle and cover portions. The gusset 90 may also induce stresses in the frangible portion of the cap 58 which permit easier removal of the cover 82.

Turning now to Figure 8, a side elevation of a cap 58 according to this invention is shown. As can best be seen in Figure 8, the handle 84 of this embodiment has ribs 88 and gussets 90 on both a top side and a bottom side. Additionally, the cap includes a cylindrical base portion 92 adjacent to the cover 82, and separated therefrom by a frangible portion 94. The frangible section 94 is a thinned section of the cap 58, which is molded in one piece in the preferred embodiment. The cylindrical base portion 92 of the cap 58 is cemented to the cylindrical portion 46 of the housing 32. When the handle 84 is lifted, the cover 82 is pulled upward, breaking from the cylindrical base portion 92 at the frangible portion 94. The cylindrical base portion 92 of the cap 58 remains cemented to the housing 32, thus indicated that this injection site has been previously used or tampered with.

Figure 9 shows a cross-section of the cap 58, taken along line 9-9 of Figure 7, showing the construction features of the cap 58 of the preferred embodiment of the present invention.

As previously mentioned, a region between the top of the sealing plug 48 and the filter member 56 can be sterilized by a sterilizing gas, and will remain sterile due to the configuration of this invention. To this end, the multiport injection site manifold 10 is assembled according to the exploded view of Figure 2 to the assembled view of Figure 6. That is, the sealing plug 48 is seated in the cylindrical portion 46 of the housing 32, and the retaining ring 50 is formed over the top of the outer periphery of the sealing plug 48, forming a fluid-tight seal between the parenteral fluid passage 74 and the sealing plug 48. The filter member 56 is then placed into the cap 58, and may be peripherally sealed therein, and the cylindrical base 92 of the cap 58 is cemented to the cylindrical portion 46 of the housing 32.

A sterilizing gas such as ethylene oxide gas, is introduced as is known in the art, into the openings 86 of the cover 82 of the cap 58. The sterilized fluid permeates the filter member 56, and kills any microbes between the top of the sealing plug 48 and the filter member 56 to create a sterile region. After the sterilizing gas is removed, the filter prevents new airborne microbes from entering into the area between the filter member 56 and the top of the sealing plug 48 until the cover 82 of the cap 58 is removed.

The manifold 10 of this invention is preferably located on the medical fluid administration path in a location that is convenient to the staff, for example, near an infusion pump. The manifold may be an integral part of a medical fluid administration set or may be a separate add-on device with appropriate tapered Luer slip (friction fit) or Luer-lock (threaded engagement) fittings for interconnection with standard medical fittings on fluid administration apparatus. When it is necessary to inject a supplemental medicament or add a supplemental fluid to the parenteral fluid stream, the user, as shown in Figure 10, lifts handle 84 on cap 58, and peels the cover 82 off of the sealing plug 48 by breaking the cover 82 from the cylindrical base portion 92 at the frangible section 94. The filter member 56 comes off with the cover 82. The user thus knows that the injection site is sterile and not previously used because the cover 82 of the cap 58 was intact.

Turning now to Figure 11, the user may then administer a supplemental medication by pushing a medicinal delivery needle 96 (shown in phantom lines), as is well known in the art, into the sealing plug 48, until the needle has penetrated beneath the sealing plug 48, and into the parenteral stream in the medical fluid passage 74. The supplemental medicament will then mix with the parenteral fluid and continue into the patient-recipient.

It should be understood that various changes and modifications to the presently preferred embodiment described herein will be apparent to those skilled in the art. The above has been offered for illustrative purposes only, and is not intended to limit the invention of this application, which is defined in the claims below.

## Claims

1. An apparatus for introducing supplemental medicament fluids into a medical fluid in a medical fluid administration system, the apparatus comprising:
a housing having an inlet and an outlet for said medical fluid and at least one aperture defining a supplemental fluid passage, said inlet, said outlet and said at least one aperture being in fluid communication;
sealing means cooperatively associated with said aperture for sealing said supplemental fluid passage;
covering means removably affixed to said housing and covering said at least one aperture and said sealing means;
a microporous filter member interposed between said sealing means and said covering means, said filter member being gas permeable and substantially microbe impermeable;
said covering means defining a fluid communication passageway with said filter member from without said covering means.

2. An apparatus as recited in Claim 1 wherein said covering means comprises a cap, said cap having a first portion covering at least part of said aperture, a second portion affixed to said housing and a frangible portion joining said first portion and said second portion.

3. An apparatus as recited in Claim 2 wherein said cap includes a handle for grasping to remove said first portion.

4. An apparatus as recited in Claim 1 wherein said sealing means comprises a pierceable, self-sealing plug.

5. An apparatus as recited in Claim 1 wherein said housing is formed of a thermoplastic material.

6. An apparatus as recited in Claim 2 wherein said cap is formed of a thermoplastic material.

7. An apparatus as recited in Claim 1 wherein said housing is of unitary construction.

8. An apparatus as recited in Claim 1 wherein said cover is tamper indicative.

9. An apparatus as recited in Claim 2 wherein said cap is tamper indicative.

10. An apparatus for administering medical fluids, the apparatus comprising:
means for accessing the contents of said medical solution container;
a first tube in fluid communication with said accessing means;
an injection site for introducing supplemental medicaments in fluid communication with said first tube, said injection site including:
a housing having an inlet, an outlet and
a passage defining a medical fluid passage connecting said inlet and said outlet, and at least one aperture defining a supplemental fluid passage, said medical fluid passage and said at least one aperture being in fluid communication, and said inlet being in fluid communication with said first tube;
sealing means cooperatively associated with said aperture for sealing said supplemental fluid passage;
covering means removably affixed to said housing and covering said at least one aperture and said sealing means;
a microporous filter member interposed between said sealing means and said covering means, said filter member being gas permeable and substantially microbe impermeable;
said covering means defining a fluid communication passageway with said filter member from without said covering means; and
a second tube in fluid communication with said outlet of said manifold, said second tube including connecting means for connecting said apparatus to a patient.

11. An apparatus as recited in Claim 10 wherein said covering means comprises a cap, said cap having a first portion covering at least part of said aperture, a second portion affixed to said housing and a frangible portion joining said first portion and said second portion.

12. An apparatus as recited in Claim 11 wherein said cap includes a handle for grasping to remove said first portion.

13. An apparatus for introducing supplemental medicament fluids into a medical fluid stream, the apparatus comprising:

a unitary housing having an inlet and an outlet for said medical fluid stream and a plurality of apertures defining supplemental fluid passages;

a sealing plug disposed within each of said plurality of apertures for sealing said supplemental fluid passage, said sealing plug being pierceable and self-sealing;

a cap covering each of said plurality of apertures, said cap having a first portion covering at least part of said aperture, a second portion affixed to said housing and a frangible portion joining said first portion and said second portion;

a microporous filter interposed between each respective sealing plug and cap, said filter being gas permeable and substantially microbe impermeable; and

each of said caps including a fluid communication passageway with said filters from without said caps.

14. An apparatus as recited in Claim 13 wherein said plurality of apertures comprises at least four apertures.

FIG. 1

FIG. 2

FIG. 3

FIG. 4

FIG. 5

FIG. 6

FIG. 7

58

82

84

9

9

90

86

88

FIG. 9

82

94

58

92

90

88

90

88

84

FIG. 8

82

58

90

88

94

84

92

88

FIG. 11

FIG. 10

84

58

82

94

92

48

96

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,P | EP-A-0 302 752  (BAXTER INTERNATIONAL INC.) <br> * Figures 4,5A-5F; page 8, line 1 - page 9, line 11 * | 1,4,7, 10 | A 61 M  39/02 |
| A,P | | 5,6,13, 14 | |
| | --- | | |
| Y | US-A-3 952 902  (PROUTY et al.) <br> * Figures 4,5,7,8; column 2, lines 43-54; column 7, lines 32-46 * | 1,4,7, 10 | |
| A | | 13 | |
| | --- | | |
| A,D | GB-A-2 084 878  (BAXTER TRAVENOL LABORATORIES INC.) <br> * Figures 1,3,6; page 1, line 118 - page 2, line 10; page 2, lines 31-34 * | 2,3,8,9 ,11,12, 13 | |
| | --- | | |
| A | US-A-3 326 401  (DE LONG) <br> * Figures 2,3; column 3, lines 27-29 * | 1 | |
| | --- | | |
| A | EP-A-0 096 115  (U.S. CLINICAL PRODUCTS, INC.) <br> * Figures 1,2,5; page 14, line 25 - page 15, line 9; page 15, lines 26-29 * | 1 | TECHNICAL FIELDS SEARCHED (Int. Cl.5) <br><br> A 61 M <br> A 61 J <br> B 65 D |

The present search report has been drawn up for all claims

| Place of search | Date of completion of the search | Examiner |
|---|---|---|
| THE HAGUE | 05-04-1990 | SEDY, R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)